# EUROPEAN PATENT APPLICATION

(11) **EP 4 851 174 A1**
(43) Date of publication of application: **09.12.2026**
(21) Application number: 24921228.3
(22) Date of filing: 21.05.2024
(51) Int. Cl.: C07H 19/048, A61K 8/60, A61Q 5/02, A61Q 19/08, A61Q 19/00, A23L 33/13, A61P 29/00, A61P 15/00, A61P 27/06, A61P 25/00, A61P 39/02, A61P 9/04, A61P 9/00, A61P 1/16, A61P 13/12, A61P 17/14, A61P 27/02, A61P 21/00, A61P 3/10

(54) **REDUCED BETA-NICOTINAMIDE MONONUCLEOTIDE SALT AND COMPOSITION THEREOF, PREPARATION METHOD THEREFOR AND USE THEREOF**

(30) Priority: 02.02.2024 CN 202410148829
(71) Applicant: EffePharm (Shanghai) Co., Ltd., Shanghai 201601 (CN)
(72) Inventor: GAO, Xing, Shanghai 201601 (CN); YU, Jianjun, Shanghai 201601 (CN); SHEN, Qiang, Shanghai 201601 (CN); XU, Qinyuan, Shanghai 201601 (CN); DING, Yunkang, Shanghai 201601 (CN); LING, Debin, Shanghai 201601 (CN); XUAN, Lulu, Shanghai 201601 (CN); LI, Yang, Shanghai 201601 (CN); LIU, Jialing, Shanghai 201601 (CN)
(74) Representative: Lavoix
(86) International application number: PCT/CN2024/094490
(87) International publication number: WO 2025/161186

(57) **Abstract**

Provided is a reduced β-nicotinamide mononucleotide salt-type compound represented by formula (I), and particularly provided are a reduced β-nicotinamide mononucleotide salt and a composition thereof, a preparation method therefore, and a use thereof. The salt-type compound may serve as an active ingredient in a pharmaceutical composition for enhancing NAD⁺ to delay aging and/or for preventing or treating diseases or symptoms induced or caused by NAD⁺ deficiency and for preventing and/or treating diseases or symptoms induced or caused by deficiencies in essential elements and/or essential amino acids of the human body. In addition, compared with NMNH disodium salt crystalline and amorphous forms, the reduced β-nicotinamide mononucleotide salt-type compound exhibits long-term stability and moisture absorption resistance and is more beneficial to long-term storage and market promotion.

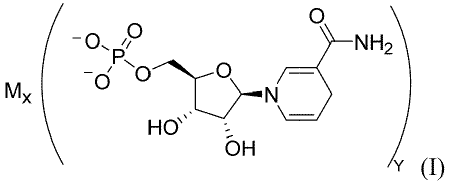

## Description

### Technical Field

The present invention relates to the field of chemical raw materials for pharmaceuticals, health products, cosmetics, and food additives, and specifically relates to reduced β-nicotinamide mononucleotide salts and composition thereof, preparation method therefor and use thereof.

### Background Art

As one of the most popular molecules in the anti-aging field, nicotinamide adenine dinucleotide (NAD⁺) has invariably become the center of anti-aging substances throughout generations. NAD⁺ is an important coenzyme required for more than 500 enzymatic reactions and is well known for its role in oxidation and reduction (*Ansari and Raghava, 2010*; *Rajman et al., 2018; Stein and Imai, 2012*). Increasing studies indicate that elevating NAD⁺ equivalents can significantly improve the functions of multiple organs, including liver function, renal function, cardiac function, and skeletal muscle function (*Canto et al., 2012; Mills et al., 2016; Rajman et al., 2018*). NAD⁺ can be synthesized via the de novo biosynthesis pathway using tryptophan, via the Preiss-Handler pathway using nicotinic acid (NA), and via the salvage pathway using nicotinamide (NAM), nicotinamide riboside (NR), and nicotinamide mononucleotide (NMN) (*Canto et al., 2015*; *Chiarugi et al., 2012*; *Johnson and Imai, 2018*). In particular, as key NAD⁺ intermediates, NAM, NR, and NMN have been extensively studied for their potential therapeutic effects in many mouse disease models (*Mills et al., 2016*), among which NMN is considered the most suitable NAD⁺ precursor at present and is currently selling well in the global market, highly favored by consumers.

NMNH (molecular structure as shown in formula (A)), whose Chinese name is "reduced nicotinamide mononucleotide" or "reduced β-nicotinamide mononucleotide," is the reduced form of NMN, a new precursor for NAD⁺ supplementation, which exhibits better NAD⁺ promoting effect than NMN and other biological functions such as increasing cellular antioxidant capacity, reducing fat accumulation, reducing inflammatory responses, and inhibiting tumor cell growth. It is a health-promoting agent with significant commercial potential (WO2021098725A1).

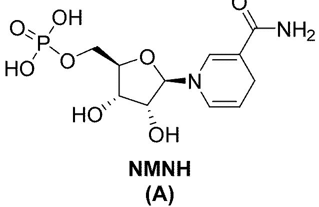

Human elements refer to the chemical elements that compose the human body. The human body is composed of chemical elements, with more than 60 types of elements present. In the elemental composition of a standard healthy adult, magnesium (Mg) accounts for 0.05% and potassium (K) accounts for 0.35%, both exceeding 0.01%, thus belonging to the essential macro-elements of the human body. Zinc (Zn), iron (Fe), copper (Cu), and manganese (Mn) are essential trace elements for the human body. Deficiency of these elements may affect human growth and development, and may even induce various clinical symptoms and diseases.

D-glucosamine, also known as glucosamine, and 2-deoxy-glucosamine, is a common amino sugar and a natural substance, which stimulates the human body to produce glycosaminoglycans for the repair and formation of cartilage and is commonly used in the treatment of osteoarthritis. L-arginine is an encoding amino acid during protein synthesis and one of the eight essential amino acids for humans. L-lysine is one of the essential amino acids for humans, which promotes human development, enhances immune function, and improves the function of the central nervous system. L-histidine is a semi-essential amino acid for humans, used for the treatment of gastric ulcers, anemia, and cardiovascular diseases such as angina pectoris, aortitis, and cardiac insufficiency.

NMNH is the reduced form of NMN, which is air-sensitive, readily oxidized, and unstable, making it unfavorable for long-term storage and commercial promotion. WO2023160405 (A1) reported the NMNH disodium salt-type compound and its crystalline forms and amorphous form. No literature has reported other salt forms.

Furthermore, the current application of NMNH is relatively limited and cannot provide complementary effects with other human essential elements or compounds.

Therefore, there is an urgent need in the art to develop new salt forms that improve product stability for convenient commercial promotion, and that can also supplement different human essential elements and essential amino acids.

### Summary of the Invention

The purpose of the present invention is to develop a new NMNH salt form with good product stability, being convenient for storage and commercial promotion, and capable of supplement different human essential elements and essential amino acids, specifically relating to a reduced β-nicotinamide mononucleotide salt and composition thereof, preparation method therefor and use thereof.

In a first aspect of the present invention, provided is a reduced β-nicotinamide mononucleotide salt-type compound (NMNH-M) represented by formula (I): 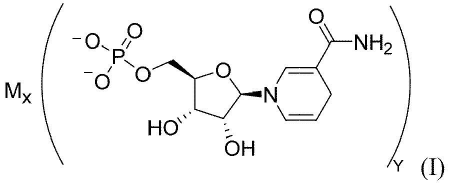 wherein,
M is independently selected from the group consisting of: Mg²⁺, Zn²⁺, Fe³⁺, Cu²⁺, Mn²⁺, and an organic matter;
when M is Mg²⁺, X=1, Y=1;
when M is Zn²⁺, X=1, Y=1;
when M is Fe³⁺, X=2, Y=3;
when M is Cu²⁺, X=1, Y=1;
when M is Mn²⁺, X=1, Y=1;
whrein the organic matter is selected from the group consisting of: D-glucosamine, L-arginine, L-lysine and L-histidine.

In another preferred embodiment, M is further selected from metal ions of calcium and selenium.

In another preferred embodiment, when M in the compound of formula (I) is Mg²⁺, the compound of formula (I) is designated as NMNH-Mg.

In another preferred embodiment, the compound of formula (I) is selected from the group consisting of: NMNH-Mg, NMNH-Zn, NMNH-Fe(III), NMNH-Cu(II), NMNH-Mn(II), NMNH-bis(D-glucosamine), NMNH-D-glucosamine, NMNH-bis(L-arginine), NMNH-L-arginine, NMNH-bis(L-lysine), NMNH-L-lysine, NMNH-bis(L-histidine), and NMNH-L-histidine.

In another preferred embodiment, the compound is amorphous.

In another preferred embodiment, M is Mg²⁺, X=1, Y=1.

In a second aspect of the present invention, provided is a method for preparing the compound according to the first aspect, comprising steps of:
(1) adding reduced β-nicotinamide mononucleotide disodium salt to a first solvent, stirring and adjusting pH to 3-4 to obtain solution A, or to generate *in situ* reduced β-nicotinamide mononucleotide solution A through a reaction;
(2) adding a solution of an inorganic salt, an organic matter, or an organic salt to solution A obtained in step (1), adjusting pH, and stirring for reaction to obtain solution B;
(3) removing the solvent from solution B obtained in step (2) and drying to obtain the reduced β-nicotinamide mononucleotide salt-type compound.

In another preferred embodiment, the first solvent is selected from the group consisting of: water, methanol, and ethanol.

In another preferred embodiment, the inorganic salt is selected from the group consisting of: MgCl₂, KCl, CaCl₂, ZnCl₂, FeCl₃, CuCl₂, MnCl₂, Mg(OH)₂, KOH, Ca(OH)₂, NaOH and Cu(OH)₂.

In another preferred embodiment, the organic salt is selected from the group consisting of: magnesium acetate, zinc acetate, D-glucosamine hydrochloride, L-arginine hydrochloride, L-lysine hydrochloride and L-histidine hydrochloride.

In another preferred embodiment, the organic matter is selected from D-glucosamine, L-arginine, L-lysine and L-histidine.

In another preferred embodiment, the molar ratio of the reduced β-nicotinamide mononucleotide to the corresponding organic matter is 1:1-2.5, preferably 1:1-2.

In another preferred embodiment, the molar ratio of the reduced β-nicotinamide mononucleotide to the inorganic salt is 1:0.5-1.1.

In another preferred embodiment, the molar ratio of the reduced β-nicotinamide mononucleotide to the organic salt is 1:0.8-2.5, preferably 1:1-2.

In a third aspect of the present invention, provided is a pharmaceutical composition, comprising the reduced β-nicotinamide mononucleotide salt-type compound according to the first aspect or a pharmaceutically acceptable carrier thereof.

In another preferred embodiment, the pharmaceutically acceptable carrier is selected from the group consisting of: saline, buffers, glucose, water, glycerol, ethanol, and combinations thereof.

In another preferred embodiment, the pharmaceutical composition is in the form of tablets, capsules, lyophilized powders, solutions, injections, dressings, and the like.

In a fourth aspect of the present invention, provided is a method for preparing a pharmaceutical composition, comprising a step of:
mixing the reduced β-nicotinamide mononucleotide salt-type compound according to the first aspect with a pharmaceutically acceptable carrier to form the pharmaceutical composition.

In a fifth aspect of the present invention, provided is a use of the reduced β-nicotinamide mononucleotide salt-type compound according to the first aspect or the pharmaceutical composition according to the third aspect for the preparation of a product for elevating nicotinamide adenine dinucleotide (NAD⁺) to delay aging, and/or for preventing or treating diseases or symptoms caused by or resulting from NAD⁺ deficiency.

In another preferred embodiment, the diseases or symptoms are selected from the group consisting of: aging-related symptoms or diseases, immunity decline-related symptoms or diseases, metabolic diseases or symptoms, and neurodegenerative changes.

In another preferred embodiment, the aging-related diseases or symptoms are selected from the group consisting of: skin tissue atrophy and collapse, dry skin, roughness, laxity, wrinkles, pellagra, lack of energy, muscle weakness, sarcopenia, easy fatigue, cognitive decline, memory decline, cognitive decline, and Alzheimer's disease.

In another preferred embodiment, the immunity decline-related diseases or symptoms are selected from the group consisting of: weakness, immune system disorders, arthritis, and cancer.

In another preferred embodiment, the metabolic diseases or symptoms are selected from the group consisting of: endocrine disorders, metabolic disturbances, diabetes, obesity, atherosclerosis, hypertension, and metabolic dysfunction.

In a sixth aspect of the present invention, provided is a use of the reduced β-nicotinamide mononucleotide salt-type compound according to the first aspect or the pharmaceutical composition according to the third aspect for the preparation of a product for preventing and/or treating diseases or symptoms caused by or resulting from deficiency of human essential elements.

In another preferred embodiment, the product is selected from the group consisting of: pharmaceuticals, dietary supplements, health products, nutritional supplements, or food additives.

In another preferred embodiment, the essential elements include magnesium, calcium, zinc, iron, copper, manganese, and selenium.

In another preferred embodiment, the diseases or symptoms are selected from the group consisting of: magnesium deficiency-related diseases or symptoms, calcium deficiency-related diseases or symptoms, zinc deficiency-related diseases or symptoms, iron deficiency-related diseases or symptoms, copper deficiency-related diseases or symptoms, manganese deficiency-related diseases or symptoms, and selenium deficiency-related diseases or symptoms.

In another preferred embodiment, the magnesium deficiency-related diseases or symptoms are selected from the group consisting of: anorexia, nausea, vomiting, weakness, apathy, memory impairment, nervous tension, irritability, confusion, restlessness, athetosis-like movements, and epileptiform seizures.

In another preferred embodiment, the calcium deficiency-related diseases or symptoms are selected from the group consisting of: muscle cramps, limb weakness, generalized pain, loose teeth, excessive sweating, easy fatigue, tracheal spasm causing dry cough without sputum, bone spurs, joint pain, sensitivity to wind, high blood pressure, constipation, dysmenorrhea, short stature, genu valgum and genu varum.

In another preferred embodiment, the zinc deficiency-related diseases or symptoms are selected from the group consisting of: growth retardation, reduced immunity, slow wound healing, dermatitis, hypogonadism, loss of appetite, taste disturbance, geophagia, delayed dark adaptation, narcolepsy, depression and stress symptoms, chronic diarrhea, alopecia, and dermatitis.

In another preferred embodiment, the iron deficiency-related diseases or symptoms are selected from the group consisting of: night terrors, lethargy, anorexia, picky eating, growth retardation, frequent dizziness, insomnia, colds, fever, cough, diarrhea, inattention, poor comprehension and memory, pallor, sallow complexion, pale lips, dull hair, insomnia with excessive dreaming, limb weakness, intolerance to cold, hypomenorrhea, amenorrhea or menorrhagia, dysmenorrhea, skin prone to wrinkles and age spots, oral ulcers, iron deficiency anemia, easy fatigue, chills, and decreased resistance.

In another preferred embodiment, the copper deficiency-related diseases or symptoms are selected from the group consisting of: fatigue and weakness caused by reduced red blood cell count (anemia), increased risk of infection caused by reduced white blood cell count, osteoporosis, and tingling and numbness in hands and feet, muscle weakness, impaired consciousness, irritability, mild depression, and impaired coordination due to nerve damage.

In another preferred embodiment, the manganese deficiency-related diseases or symptoms are selected from the group consisting of: growth and reproductive disorders, abnormal development in children, ataxia, tardive dyskinesia, osteoporosis, skeletal hypoplasia, hypercholesterolemia, easy cramping, abnormal fat metabolism, and central nervous system disorders.

In another preferred embodiment, the selenium deficiency-related diseases or symptoms are selected from the group consisting of: decreased human immune function, and in severe cases, hair loss and nail shedding; some patients may develop skin symptoms, nervous system diseases, and dental damage; long-term severe selenium deficiency may lead to Keshan disease, Kashin-Beck disease, and other serious conditions.

In another preferred embodiment, the human essential elements are selected from the group consisting of: magnesium (Mg), zinc (Zn), iron (Fe), copper (Cu), and manganese (Mn).

In another preferred embodiment, the essential elements further include calcium (Ca) and selenium (Se).

In a seventh aspect of the present invention, provided is a use of the reduced β-nicotinamide mononucleotide salt-type compound according to the first aspect or the pharmaceutical composition according to the third aspect for the preparation of a product for preventing and/or treating diseases or symptoms caused by or resulting from conditions such as malnutrition, loss of appetite, and developmental insufficiency.

In another preferred embodiment, the D-glucosamine deficiency-related diseases or symptoms are selected from the group consisting of: joint inflammation and swelling, cartilage damage, and osteoarthritis.

In another preferred embodiment, the L-arginine deficiency-related diseases or symptoms are selected from the group consisting of: congestive heart failure, cystitis, male erectile dysfunction, infertility, cardiovascular and cerebrovascular diseases, diabetes, hypertension, atherosclerosis, and angina pectoris.

In another preferred embodiment, the L-lysine deficiency-related diseases or symptoms are selected from the group consisting of: growth retardation in height, reduced immunity, inattention, fatigue and weakness, loss of appetite, osteoporosis, and hypoproteinemia.

In another preferred embodiment, the L-histidine deficiency-related diseases or symptoms are selected from the group consisting of: anemia and hemoglobin reduction, joint pain, rheumatoid arthritis, anxiety and depressive symptoms, fatigue and dizziness, inflammation of the skin and mucous membranes and dry or scaly skin lesions, poor kidney and liver function, eczema, allergies, hypertension, cognitive decline, gastric ulcers, symptoms caused by renal failure or renal dialysis, growth and development disorders in infants and young children, and heart disease.

It should be understood that within the scope of the present invention, the above technical features of the present invention and the technical features specifically described below (e.g., in the Examples) can be combined with each other to constitute new or preferred technical solutions. Due to space limitations, they are not listed one by one here.

### Description of the Drawings

Figure 1 shows the XRPD pattern of NMNH magnesium salt amorphous form.
Figure 2 shows the XRPD pattern of NMNH dipotassium salt amorphous form.
Figure 3 shows the XRPD pattern of NMNH zinc salt amorphous form.
Figure 4 shows the XRPD pattern of NMNH ferric salt amorphous form.
Figure 5 shows the XRPD pattern of NMNH ferrous salt amorphous form.
Figure 6 shows the XRPD pattern of NMNH cupric salt amorphous form.
Figure 7 shows the XRPD pattern of NMNH manganous salt amorphous form.
Figure 8 shows the ¹H NMR spectrum of NMNH inorganic salts.
Figure 9 shows the XRPD pattern of NMNH-bis(D-glucosamine) salt amorphous form.
Figure 10 shows the XRPD pattern of NMNH-D-glucosamine salt amorphous form.
Figure 11 shows the XRPD pattern of NMNH-bis(L-arginine) salt amorphous form.
Figure 12 shows the XRPD pattern of NMNH-L-arginine salt amorphous form.
Figure 13 shows the XRPD pattern of NMNH-bis(L-lysine) salt amorphous form.
Figure 14 shows the XRPD pattern of NMNH-L-lysine salt amorphous form.
Figure 15 shows the XRPD pattern of NMNH-bis(L-histidine) salt amorphous form.
Figure 16 shows the XRPD pattern of NMNH-L-histidine salt amorphous form.
Figure 17 shows the ¹H NMR spectrum of NMNH-bis(D-glucosamine) salt.
Figure 18 shows the ¹H NMR spectrum of NMNH-D-glucosamine salt.
Figure 19 shows the ¹H NMR spectrum of NMNH-bis(L-arginine) salt.
Figure 20 shows the ¹H NMR spectrum of NMNH-L-arginine salt.
Figure 21 shows the ¹H NMR spectrum of NMNH-bis(L-lysine) salt.
Figure 22 shows the ¹H NMR spectrum of NMNH-L-lysine salt.
Figure 23 shows the ¹H NMR spectrum of NMNH-bis(L-histidine) salt.
Figure 24 shows the ¹H NMR spectrum of NMNH-L-histidine salt.

### Detailed Description of the Invention

Through extensive and in-depth research, the present inventors have unexpectedly developed for the first time a reduced β-nicotinamide mononucleotide salt-type compound (NMNH-M), wherein M in the formula is independently selected from the group consisting of: Mg²⁺, Zn²⁺, Fe³⁺, Cu²⁺, and Mn²⁺. The compound of the present invention, or as an active ingredient of a pharmaceutical composition, is used for elevating NAD⁺ to delay aging, and/or for preventing or treating diseases or symptoms caused by or resulting from NAD⁺ deficiency, and can also be used for preventing and/or treating diseases or symptoms caused by or resulting from deficiency of human essential elements and/or essential amino acids. The research of the present invention demonstrates that the reduced β-nicotinamide mononucleotide salt-type compound of the present invention, compared with NMNH disodium salt crystalline forms and amorphous forms, exhibits long-term sustained stability and moisture resistance, which is more favorable for long-term storage and commercial promotion. Furthermore, the compound of the present invention meets the shelf-life requirements for commercial products and is suitable for use in pharmaceutical compositions, health products, cosmetics, food additives, and the like. On this basis, the inventors have completed the present invention.

### Terms

As used herein, the term "NMNH" has the name "reduced nicotinamide mononucleotide" or "reduced β-nicotinamide mononucleotide," which is the reduced form of β-NMN and a new precursor for NAD⁺ supplementation.

As used herein, "NMNH," "reduced nicotinamide mononucleotide," and salts thereof all refer to the "β-configuration" thereof.

In the present invention, in the compound of formula (I):
when M is Mg²⁺, the compound of formula (I) is designated as NMNH-Mg;
when M is Ca²⁺, the compound of formula (I) is designated as NMNH-Ca;
when M is K¹⁺, the compound of formula (I) is designated as NMNH-K₂;
when M is Na¹⁺, the compound of formula (I) is designated as NMNH-Na₂;
when M is Zn²⁺, the compound of formula (I) is designated as NMNH-Zn;
when M is Fe³⁺, the compound of formula (I) is designated as NMNH-Fe(III);
when M is Fe²⁺, the compound of formula (I) is designated as NMNH-Fe(II);
when M is Cu²⁺, the compound of formula (I) is designated as NMNH-Cu(II);
when M is Mn²⁺, the compound of formula (I) is designated as NMNH-Mn(II);
when M is Se²⁺, the compound of formula (I) is designated as NMNH-Se(II).

As used herein, the terms "reduced β-nicotinamide mononucleotide salt-type compound," "NMNH-M", and "compound of the present invention" are used interchangeably. The NMNH-M of the present invention is a salt formed from reduced β-nicotinamide mononucleotide and the corresponding metal ion or organic matter. Unless otherwise specified, the "compound of the present invention" herein refers to a compound of the β-configuration.

### Preparation Method of Reduced β-Nicotinamide Mononucleotide Salt-type compound

In the present invention, the preparation method of the compound comprises steps of:
(1) adding reduced β-nicotinamide mononucleotide disodium salt to a first solvent, stirring and adjusting pH to 3-4 to obtain solution A, or to *in situ* generate reduced β-nicotinamide mononucleotide solution A through a reaction;
(2) adding a solution of an inorganic salt, an organic matter, or an organic salt to solution A obtained in step (1), adjusting pH, and stirring for reaction to obtain solution B;
(3) removing the solvent from solution B obtained in step (2) and drying to obtain the reduced β-nicotinamide mononucleotide salt-type compound.

Preferably, the first solvent is selected from the group consisting of: water, methanol, and ethanol.

Preferably, the inorganic salt is selected from the group consisting of: MgCl₂, KCl, CaCl₂, ZnCl₂, FeCl₃, CuCl₂, MnCl₂, Mg(OH)₂, KOH, Ca(OH)₂, NaOH, and Cu(OH)₂.

Preferably, the organic matter is selected from the group consisting of: D-glucosamine, L-arginine, L-lysine, and L-histidine.

Preferably, the organic salt is selected from the group consisting of: magnesium acetate, zinc acetate, D-glucosamine hydrochloride, L-arginine hydrochloride, L-lysine hydrochloride, and L-histidine hydrochloride.

### Pharmaceutical Composition

The reduced β-nicotinamide mononucleotide salt-type compound of the present invention can be used for elevating NAD⁺ to delay aging, and/or for preventing or treating diseases or symptoms caused by or resulting from NAD⁺ deficiency. Generally, the reduced β-nicotinamide mononucleotide salt-type compound of the present invention can be formulated in non-toxic, inert, and pharmaceutically acceptable aqueous carrier media. The formulated pharmaceutical composition can be administered by conventional routes, including but not limited to: intramuscular, intraperitoneal, intravenous, subcutaneous, intradermal, or topical administration.

The reduced β-nicotinamide mononucleotide salt-type compound of the present invention can be used directly for the treatment of diseases, such as diseases or symptoms caused by or resulting from NAD⁺ deficiency, and diseases or symptoms caused by or resulting from deficiency of human essential elements and essential amino acids.

Preferably, the human essential elements are selected from the group consisting of: magnesium (Mg), calcium (Ca), potassium (K), zinc (Zn), iron (Fe), copper (Cu), manganese (Mn), and selenium (Se).

Preferably, the amino acids are selected from the group consisting of: L-arginine, L-lysine, and L-histidine.

The organic matter in the reduced β-nicotinamide mononucleotide organic salt-type compound is selected from the group consisting of: D-glucosamine, L-arginine, L-lysine, and L-histidine.

The present invention also provides a pharmaceutical composition, wherein the pharmaceutical composition is formed by mixing the reduced β-nicotinamide mononucleotide salt-type compound represented by formula (I) with a pharmaceutically acceptable carrier.

The pharmaceutical composition of the present invention contains a safe and effective amount (e.g., 0.001-99.9 wt%, more preferably 0.01-90 wt%) of the reduced β-nicotinamide mononucleotide salt-type compound of the present invention and a pharmaceutically acceptable carrier or excipient.

A preferred pharmaceutical composition comprises one or more of NMNH-Na₂, NMNH-Mg, NMNH-Ca, NMNH-Zn, NMNH-Fe(III), NMNH-Cu(II), NMNH-Mn(II), NMNH-bis(D-glucosamine), NMNH-D-glucosamine, NMNH-bis(L-arginine), NMNH-L-arginine, NMNH-bis(L-lysine), NMNH-L-lysine, NMNH-bis(L-histidine), and NMNH-L-histidine, wherein Na content is ≤ 3000 mg, K content ≤ 2400 mg, Mg content ≤ 400 mg, Ca content ≤ 1000 mg, Zn content ≤ 15.0 mg, Fe content ≤ 20.0 mg, Cu content ≤ 1.5 mg, Mn content ≤ 4.0 mg, D-glucosamine content ≤ 1500 mg, L-arginine content ≤ 4000 mg, L-lysine content ≤ 2500 mg, and L-histidine content ≤ 20 mg.

More preferably, in the composition, Na content is ≤ 138 mg, K content ≤ 234 mg, Mg content ≤ 73 mg, Ca content ≤ 120 mg, Zn content ≤ 15.0 mg, Fe content ≤ 20.0 mg, Cu content ≤ 1.5 mg, Mn content ≤ 4.0 mg, D-glucosamine content ≤ 1000 mg, L-arginine content ≤ 1000 mg, L-lysine content ≤ 870 mg, and L-histidine content ≤ 20 mg.

More preferably, in the composition, Na content is ≤ 83 mg, K content ≤ 140 mg, Mg content ≤ 44 mg, Ca content ≤ 72 mg, Zn content ≤ 15.0 mg, Fe content ≤ 20.0 mg, Cu content ≤ 1.5 mg, Mn content ≤ 4.0 mg, D-glucosamine content ≤ 640 mg, L-arginine content ≤ 620 mg, L-lysine content ≤ 520 mg, and L-histidine content ≤ 20 mg.

More preferably, in the composition, Na content is 55-83 mg, K content is 94-140 mg, Mg content is 29-44 mg, Ca content is 48-72 mg, Zn content is 0.8-15.0 mg, Fe content is 1.5-20.0 mg, Cu content is 0.1-1.5 mg, Mn content is 0.3-4.0 mg, D-glucosamine content is 200-640 mg, L-arginine content is 200-620 mg, L-lysine content is 170-520 mg, and L-histidine content is 6-15 mg.

Such carriers include but are not limited to: saline, buffers, glucose, water, glycerol, ethanol, and combinations thereof. The pharmaceutical formulation should be compatible with the mode of administration. The pharmaceutical composition of the present invention can be prepared in the form of injections, for example, by conventional methods using physiological saline or aqueous solutions containing glucose and other adjuvants. Pharmaceutical compositions such as tablets and capsules can be prepared by conventional methods. Pharmaceutical compositions such as injections, solutions, tablets, and capsules are preferably manufactured under sterile conditions. The dosage of the active ingredient is a therapeutically effective amount, for example, about 1 µg/kg body weight to about 5 mg/kg body weight per day.

In addition, the pharmaceutical composition of the present invention may or may not contain other chemical reagents without pharmacological effects as compounding ingredients such as pH adjusters, stabilizers, and solubilizers.

The pharmaceutical composition of the present invention can be prepared in unit dosage forms or multiple dosage forms, and can be administered alone or in combination, and can be applied in agents and/or drug potentiators for diseases or symptoms caused by or resulting from NAD⁺ deficiency.

As used herein, the term "treatment" refers to the administration of a compound of the present invention thereby alleviating a disease or condition in a host. Thus, the term "treatment" includes preventing the occurrence of a condition in a host, particularly when the host is predisposed to the disease but has not yet been diagnosed with the disease; inhibiting the condition; and/or alleviating or curing the condition. Where the method of the present invention is used for the prevention of a condition, it should be understood that the term "prevention" does not require complete inhibition of the disease. As used herein, the term "prevention" includes the ability of those skilled in the art to identify a population susceptible to a disease, such that the compound of the present invention can be administered before the disease begins to manifest. This term does not imply that the condition is completely avoided. The compounds identified by the screening method of the present invention can be used in synergy with other compounds.

### Advantages of The Present Invention Compared with the Prior Art:

(1) The compound of the present invention can be used for elevating NAD⁺ to delay aging, and/or for preventing or treating diseases or symptoms caused by or resulting from NAD⁺ deficiency, and can also be used for preventing and/or treating diseases or symptoms caused by or resulting from deficiency of human essential elements and essential amino acids.
(2) Compared with NMNH disodium salt crystalline forms and amorphous solid forms, the reduced β-nicotinamide mononucleotide salt-type compound of the present invention exhibits better stability, lower hygroscopicity, and is more favorable for long-term storage and commercial promotion.
(3) The preparation method of the reduced β-nicotinamide mononucleotide salt-type compound of the present invention is simple and suitable for industrial production.
(4) The reduced β-nicotinamide mononucleotide salt-type compound of the present invention can be used in pharmaceutical compositions, health products, cosmetics, food additives, and the like.

The present invention is further described below in conjunction with specific examples. It should be understood that these examples are merely illustrative of the present invention and are not intended to limit the scope of the present invention. Experimental methods for which specific conditions are not indicated in the following examples are generally carried out under conventional conditions or under conditions recommended by the manufacturer. Unless otherwise indicated, percentages and parts are calculated by weight.

### Example 1. Preparation of NMNH Magnesium Salt

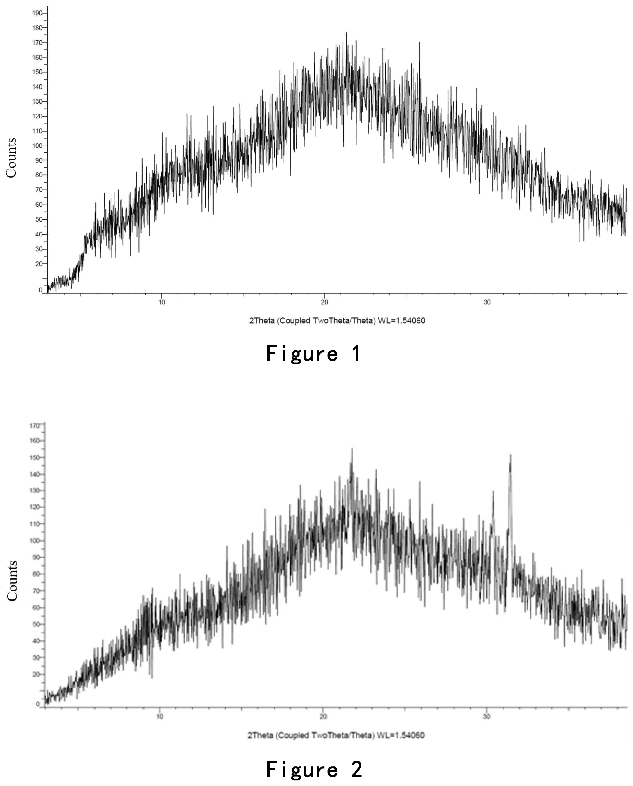

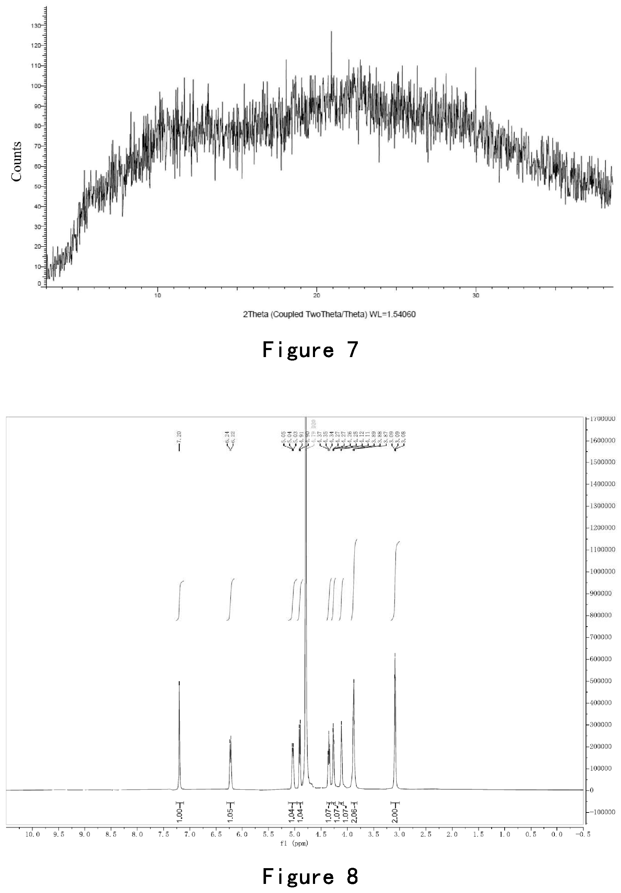

To 10 L of saturated aqueous sodium bicarbonate solution, 1.7 kg of β-NMN and 0.94 kg of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 0.30 kg of magnesium hydroxide was added; alternatively, 0.49 kg of magnesium chloride and 0.83 kg of ammonium chloride were added, and the pH of the solution was adjusted to 10 with sodium hydroxide. The resulting aqueous solution of NMNH magnesium salt was added dropwise to 20 L of stirred ethanol to precipitate the NMNH magnesium salt solid. The precipitate was filtered, and the filter cake was washed with a mixed solvent (1 L water mixed with 2 L ethanol). The filter cake was dried under vacuum at 20-30°C to obtain 1.51 kg of NMNH magnesium salt as a yellow solid, with a yield of 82.8%. HPLC purity was 99.6%, water content was 0.8%, Mg content was 6.7% by weight. XRPD analysis showed it is an amorphous solid, with the XRPD pattern shown in Figure 1 and the ¹H NMR spectrum shown in Figure 8.

### Example 2. Preparation of NMNH Dipotassium Salt

To 10 L of saturated aqueous sodium bicarbonate solution, 1.7 kg of β-NMN and 0.94 kg of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 0.57 kg of potassium hydroxide was added; alternatively, 0.76 kg of potassium chloride was added, and the pH of the solution was adjusted to 10 with aqueous ammonia. The resulting aqueous solution of NMNH dipotassium salt was added dropwise to 30 L of stirred ethanol to precipitate the NMNH dipotassium salt solid. The precipitate was filtered, and the filter cake was washed with a mixed solvent (1 L water mixed with 3 L ethanol). The filter cake was dried under vacuum at 20-30°C to obtain 1.57 kg of NMNH dipotassium salt as a yellow solid, with a yield of 75.3%. HPLC purity was 99.7%, water content was 3.8%, K content was 18.9% by weight. XRPD analysis showed it is an amorphous solid, with the XRPD pattern shown in Figure 2.

### Example 3. Preparation of NMNH Zinc Salt

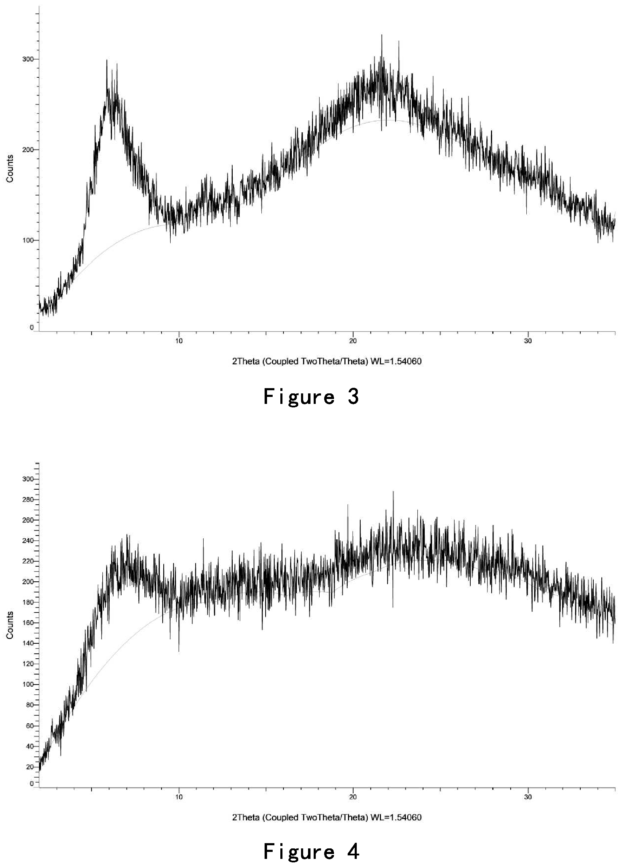

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 81.6 g of zinc chloride was added, and the pH of the solution was adjusted to 10 with aqueous ammonia to obtain an aqueous solution of NMNH zinc salt. This solution was added dropwise to 2 L of stirred methanol to precipitate the NMNH zinc salt solid. The precipitate was filtered, and the filter cake was washed with a mixed solvent (200 mL water mixed with 400 mL methanol). The filter cake was dried under vacuum at 20-30°C to obtain 214.0 g of NMNH zinc salt as a yellow solid, with a yield of 89.5%, water content was 0.2%, Zn content was 16.3% by weight. XRPD analysis showed it is an amorphous solid, with the XRPD pattern shown in Figure 3.

### Example 4. Preparation of NMNH Ferric Salt

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 64.7 g of ferric(III) chloride was added, and the pH of the solution was adjusted to 10 with aqueous ammonia to obtain an aqueous solution of NMNH ferric salt. This solution was added dropwise to 2 L of stirred methanol to precipitate the NMNH ferric salt solid. The precipitate was filtered, and the filter cake was washed with a mixed solvent (200 mL water mixed with 400 mL methanol). The filter cake was dried under vacuum at 20-30°C to obtain 199.6 g of NMNH ferric salt as a brown solid, with a yield of 89.8%, water content was 0.3%, Fe content was 10.0% by weight. XRPD analysis showed it is an amorphous solid, with the XRPD pattern shown in Figure 4.

### Example 5. Preparation of NMNH Ferrous Salt

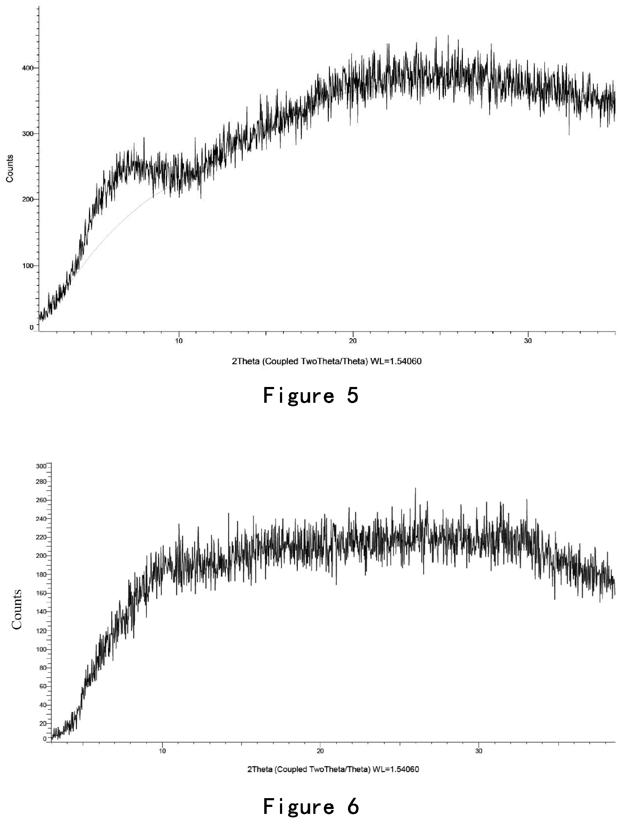

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 75.8 g of ferrous(II) chloride was added, and the pH of the solution was adjusted to 10 with aqueous ammonia to obtain an aqueous solution of NMNH ferrous salt. This solution was added dropwise to 2 L of stirred methanol to precipitate the NMNH ferrous salt solid. The precipitate was filtered, and the filter cake was washed with a mixed solvent (200 mL water mixed with 400 mL methanol). The filter cake was dried under vacuum at 20-30°C to obtain 206.3 g of NMNH ferrous salt as a pale green solid, with a yield of 88.4%, water content was 0.3%, Fe content was 14.3% by weight. XRPD analysis showed it is an amorphous solid, with the XRPD pattern shown in Figure 5.

### Example 6. Preparation of NMNH Cupric Salt

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 58.4 g of cupric(II) hydroxide was added; alternatively, 80.5 g of cupric(II) chloride was added, and the pH of the solution was adjusted to 10 with sodium hydroxide. The resulting aqueous solution of NMNH cupric salt was added dropwise to 2 L of stirred methanol to precipitate the NMNH cupric salt solid. The precipitate was filtered, and the filter cake was washed with a mixed solvent (200 mL water mixed with 400 mL methanol). The filter cake was dried under vacuum at 20-30°C to obtain 211.1 g of NMNH cupric salt as a green solid, with a yield of 88.7%, water content was 0.2%, Cu content was 15.9% by weight. XRPD analysis showed it is an amorphous solid, with the XRPD pattern shown in Figure 6.

### Example 7. Preparation of NMNH Manganous Salt

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 75.3 g of manganous(II) chloride was added, and the pH of the solution was adjusted to 10 with aqueous ammonia to obtain an aqueous solution of NMNH manganous salt. This solution was added dropwise to 2 L of stirred ethanol to precipitate the NMNH manganous salt solid. The precipitate was filtered, and the filter cake was washed with a mixed solvent (200 mL water mixed with 400 mL ethanol). The filter cake was dried under vacuum at 20-30°C to obtain 200.5 g of NMNH manganous salt as a yellow solid, with a yield of 86.1%, water content was 0.6%, Mn content was 14.1% by weight. XRPD analysis showed it is an amorphous solid, with the XRPD pattern shown in Figure 7.

### Example 8. Preparation of NMNH-bis(D-glucosamine) Salt (1:2)

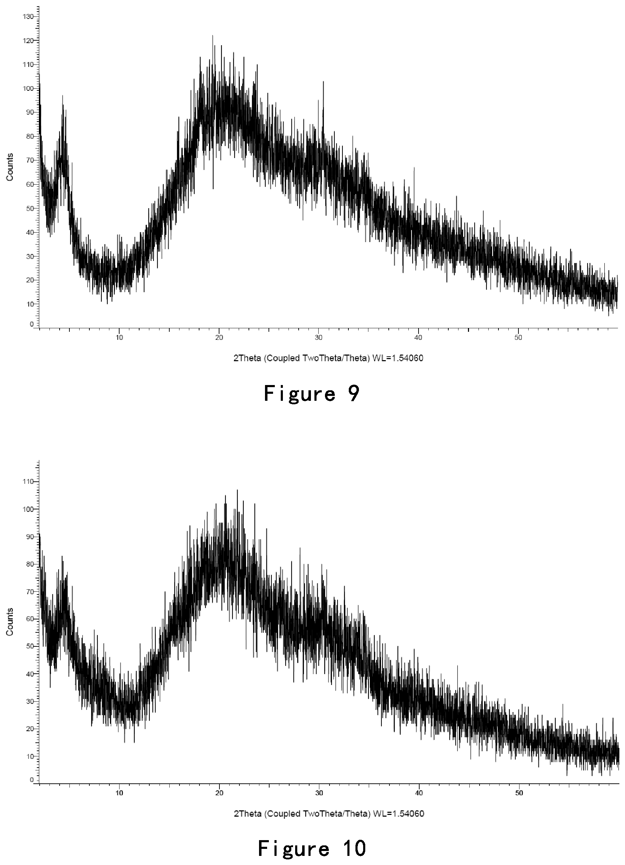

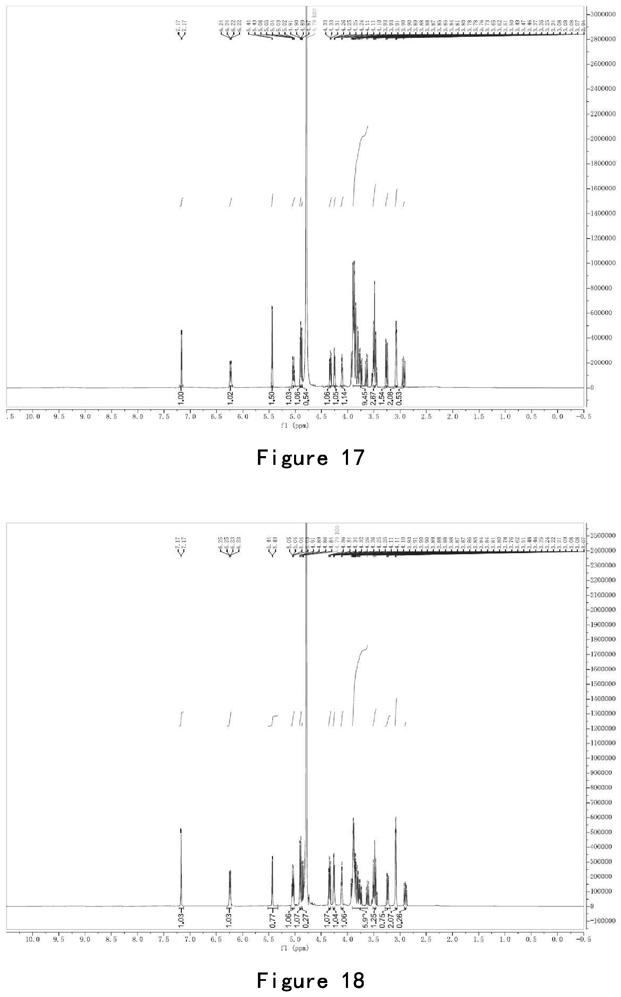

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 193.0 g of D-glucosamine was added to obtain an aqueous solution of NMNH-bis(D-glucosamine) salt. This solution was added dropwise to 2 L of stirred ethanol to precipitate the NMNH-bis(D-glucosamine) salt solid. The precipitate was filtered, and the filter cake was washed with a mixed solvent (200 mL water mixed with 400 mL ethanol). The filter cake was dried under vacuum at 20-30°C to obtain 325.0 g of NMNH-bis(D-glucosamine) salt solid, with a yield of 78.2%. XRPD analysis showed it is an amorphous solid, with the XRPD pattern shown in Figure 9 and the ¹H NMR spectrum shown in Figure 17.

### Example 9. Preparation of NMNH-D-glucosamine Salt (1:1)

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 96.5 g of D-glucosamine was added to obtain an aqueous solution of NMNH-D-glucosamine salt. This solution was added dropwise to 2 L of stirred ethanol to precipitate the NMNH-D-glucosamine salt solid. The precipitate was filtered, and the filter cake was washed with a mixed solvent (200 mL water mixed with 400 mL ethanol). The filter cake was dried under vacuum at 20-30°C to obtain 229.1 g of NMNH-D-glucosamine salt solid, with a yield of 74.3%. XRPD analysis showed it is an amorphous solid, with the XRPD pattern shown in Figure 10 and the ¹H NMR spectrum shown in Figure 18.

### Example 10. Preparation of NMNH-bis(L-arginine) Salt (1:2)

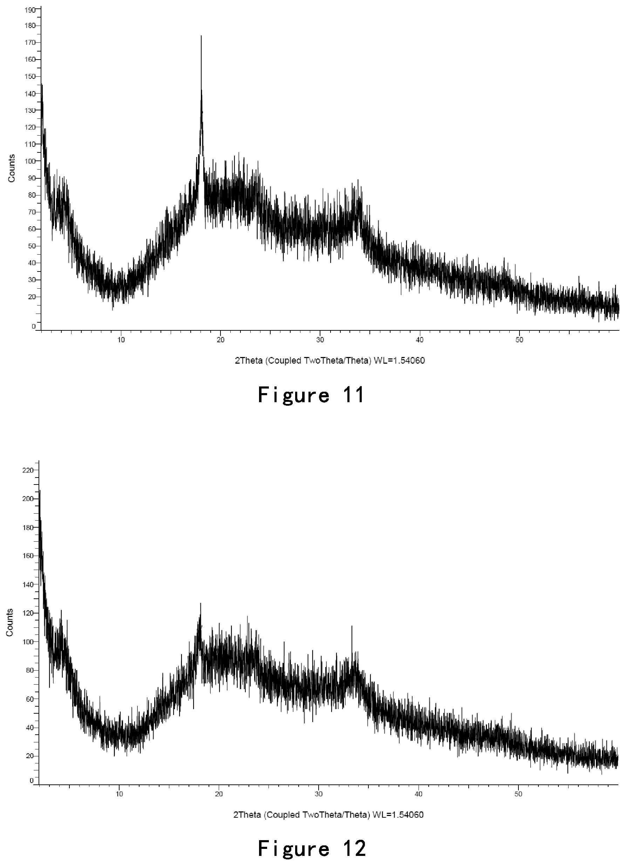

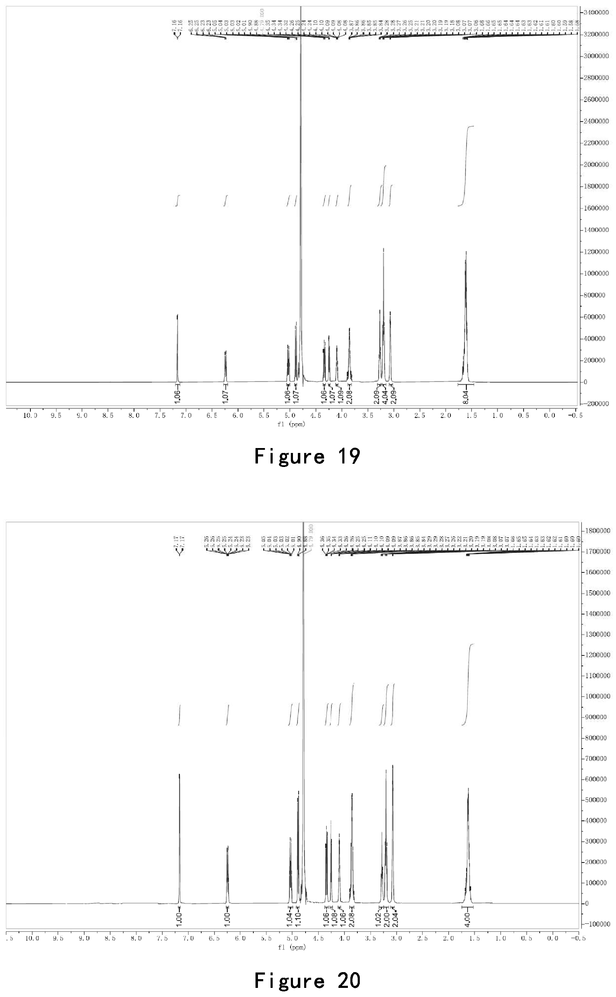

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 187.6 g of L-arginine was added to obtain an aqueous solution of NMNH-bis(L-arginine) salt. This solution was added dropwise to 2 L of stirred ethanol to precipitate the NMNH-bis(L-arginine) salt solid. The precipitate was filtered, and the filter cake was washed with a mixed solvent (200 mL water mixed with 400 mL ethanol). The filter cake was dried under vacuum at 20-30°C to obtain 260.1 g of NMNH-bis(L-arginine) salt solid, with a yield of 63.5%. XRPD analysis showed it is an amorphous solid, with the XRPD pattern shown in Figure 11 and the ¹H NMR spectrum shown in Figure 19.

### Example 11. Preparation of NMNH-L-arginine Salt (1:1)

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 93.8 g of L-arginine was added to obtain an aqueous solution of NMNH-L-arginine salt. This solution was added dropwise to 2 L of stirred ethanol to precipitate the NMNH-L-arginine salt solid. The precipitate was filtered, and the filter cake was washed with a mixed solvent (200 mL water mixed with 400 mL ethanol). The filter cake was dried under vacuum at 20-30°C to obtain 200.7 g of NMNH-L-arginine salt solid, with a yield of 65.7%. XRPD analysis showed it is an amorphous solid, with the XRPD pattern shown in Figure 12 and the ¹H NMR spectrum shown in Figure 20.

### Example 12. Preparation of NMNH-bis(L-lysine) Salt (1:2)

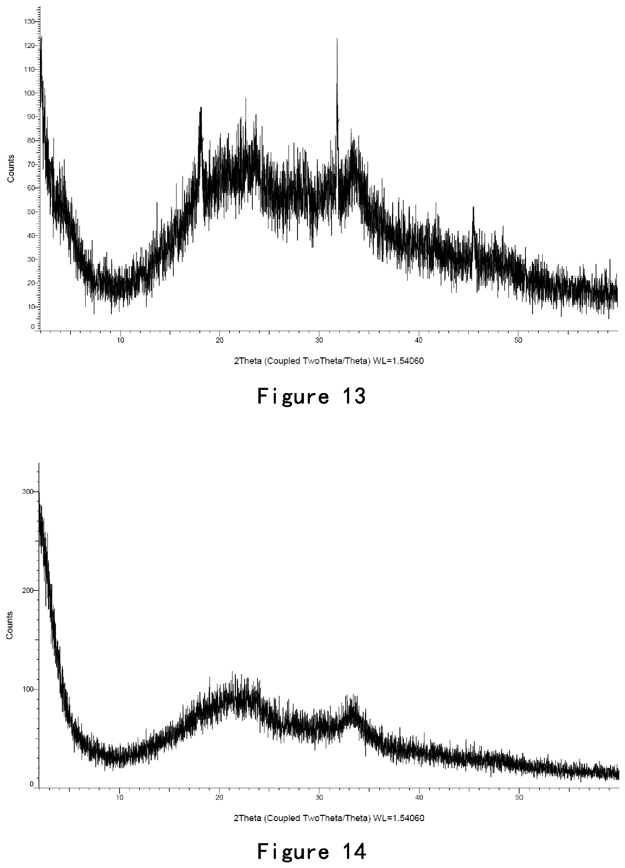

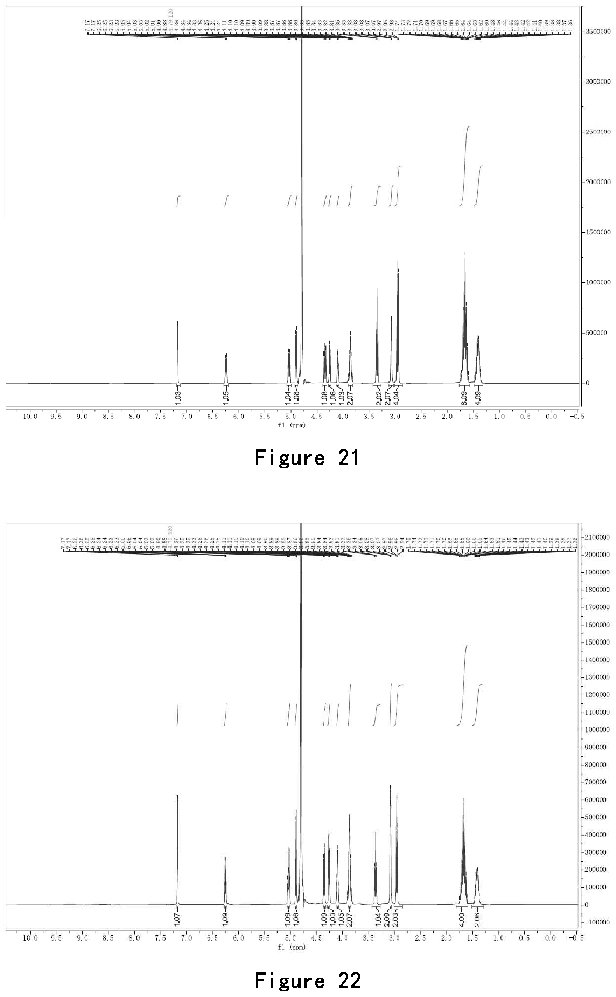

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 157.5 g of L-lysine was added to obtain an aqueous solution of NMNH-bis(L-lysine) salt. This solution was added dropwise to 2 L of stirred ethanol to precipitate the NMNH-bis(L-lysine) salt solid. The precipitate was filtered, and the filter cake was washed with a mixed solvent (200 mL water mixed with 400 mL ethanol). The filter cake was dried under vacuum at 20-30°C to obtain 259.9 g of NMNH-bis(L-lysine) salt solid, with a yield of 69.1%. XRPD analysis showed it is an amorphous solid, with the XRPD pattern shown in Figure 13 and the ¹H NMR spectrum shown in Figure 21.

### Example 13. Preparation of NMNH-L-lysine Salt (1:1)

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 78.7 g of L-lysine was added to obtain an aqueous solution of NMNH-L-lysine salt. This solution was added dropwise to 2 L of stirred ethanol to precipitate the NMNH-L-lysine salt solid. The precipitate was filtered, and the filter cake was washed with a mixed solvent (200 mL water mixed with 400 mL ethanol). The filter cake was dried under vacuum at 20-30°C to obtain 197.5 g of NMNH-L-lysine salt solid, with a yield of 68.4%. XRPD analysis showed it is an amorphous solid, with the XRPD pattern shown in Figure 14 and the ¹H NMR spectrum shown in Figure 22.

### Example 14. Preparation of NMNH-bis(L-histidine) Salt (1:2)

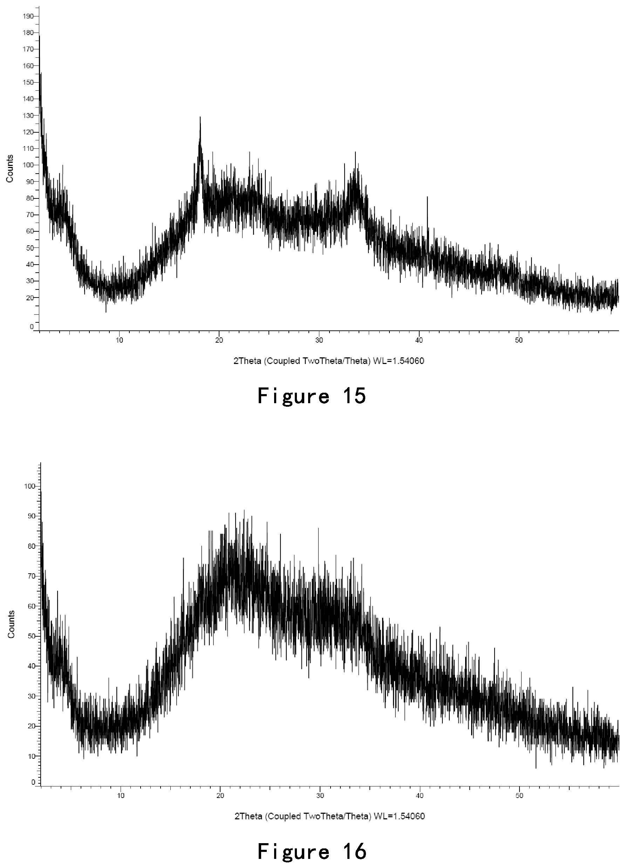

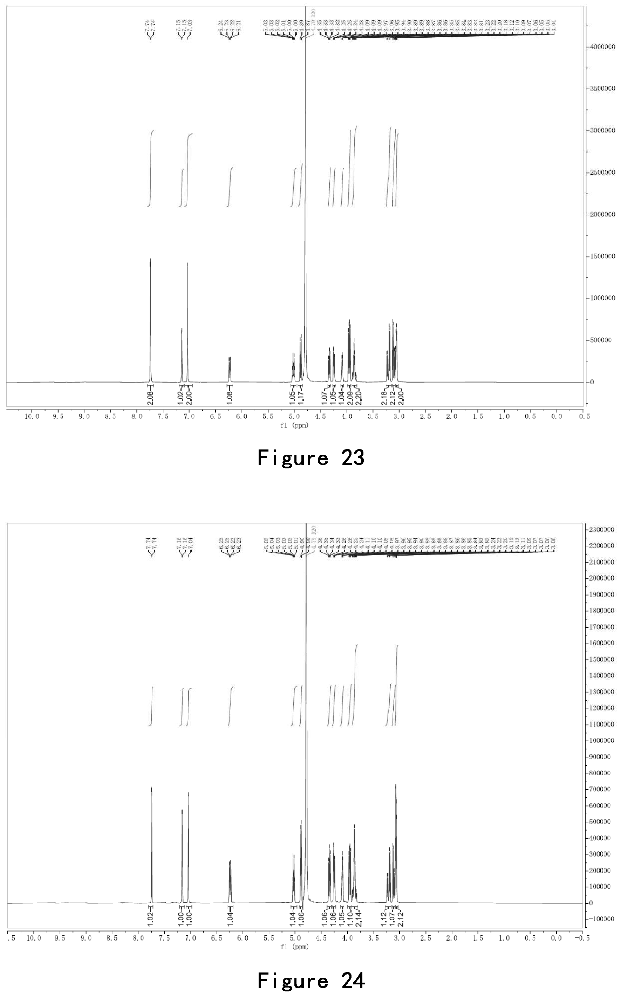

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 167.1 g of L-histidine was added to obtain an aqueous solution of NMNH-bis(L-histidine) salt. This solution was added dropwise to 2 L of stirred ethanol to precipitate the NMNH-bis(L-histidine) salt solid. The precipitate was filtered, and the filter cake was washed with a mixed solvent (200 mL water mixed with 400 mL ethanol). The filter cake was dried under vacuum at 20-30°C to obtain 277.0 g of NMNH-bis(L-histidine) salt solid, with a yield of 71.6%. XRPD analysis showed it is an amorphous solid, with the XRPD pattern shown in Figure 15 and the ¹H NMR spectrum shown in Figure 23.

### Example 15. Preparation of NMNH-L-histidine Salt (1:1)

To 1.2 L of saturated aqueous sodium bicarbonate solution, 200 g of β-NMN and 110 g of sodium dithionite were added, and the mixture was stirred at room temperature overnight. The mixture was filtered to obtain a clear solution, and the pH of the clear solution was adjusted to 3-4 with 37% hydrochloric acid. The solution was desalted by electrodialysis until the conductivity of the solution dropped to 50-100 µS. 83.6 g of L-histidine was added to obtain an aqueous solution of NMNH-L-histidine salt. This solution was added dropwise to 2 L of stirred ethanol to precipitate the NMNH-L-histidine salt solid. The precipitate was filtered, and the filter cake was washed with a mixed solvent (200 mL water mixed with 400 mL ethanol). The filter cake was dried under vacuum at 20-30°C to obtain 216.1 g of NMNH-L-histidine salt solid, with a yield of 73.5%. XRPD analysis showed it is an amorphous solid, with the XRPD pattern shown in Figure 16 and the ¹H NMR spectrum shown in Figure 24.

### Example 16. NMNH Salt Form Screening and Performance Evaluation

Accelerated studies were conducted on the performance of various NMNH salt-types, and the results are shown in Table 1 below:

**Table 1: Performance of various NMNH salt-types**

| Salt-type | Solid Form (Color) | 25°C, 65% RH, Open Placement (Days) | Results |
|---|---|---|---|
| NMNH-Na₂ (WO202316040 5A1) | Amorphous (Yellow) | 1 day | Powder absorbed moisture and turned oily; purity decreased from 99.30% to 99.02%. |
| | Crystalline Form (Pink) | 1 day / 5 days | 1 day: crystalline Form C and Form B absorbed moisture and transformed into Form A; Form A moisture content was 19%-30%; 5 days: purity decreased from 99.33% to 99.01%. |
| NMNH-K₂ | Amorphous (Yellow) | 1 day | Powder absorbed moisture and turned oily; purity decreased from 99.61% to 99.24%. |
| NMNH-Mg | Amorphous (Yellow) | 30 days | Remained yellow solid powder; purity unchanged (99.53%), moisture content unchanged (0.23%). |
| NMNH-Zn | Amorphous (Yellow) | 30 days | Remained yellow solid powder; moisture content unchanged (0.18%). |
| NMNH-Fe(III) | Amorphous (Brown) | 30 days | Remained brown solid powder; moisture content unchanged (0.21%). |
| NMNH-Fe(II) | Amorphous (Pale green) | 1 day | Solid changed from pale green to dark brown; Fe(II) was oxidized by air to Fe(III): readily oxidized by air. |
| NMNH-Cu(II) | Amorphous (Green) | 30 days | Remained green solid powder; moisture content unchanged (0.15%). |
| NMNH-Cu(I) | Product not obtained. | (No data) | (No data) |
| NMNH-Mn(II) | Amorphous (Light yellow) | 30 days | Remained green solid powder; moisture content unchanged (0.15%). |
| NMNH-bis(D-gl ucosamine) salt (1:2) | Amorphous (Yellow) | 30 days | Remained yellow solid powder; moisture content unchanged (0.48%). |
| NMNH-D-gluco samine salt (1:1) | Amorphous (Yellow) | 30 days | Remained yellow solid powder; moisture content unchanged (0.53%). |
| NMNH-bis(L-ar ginine) salt (1:2) | Amorphous (Yellow) | 30 days | Remained yellow solid powder; moisture content unchanged (0.58%). |
| NMNH-L-argini ne salt (1:1) | Amorphous (Yellow) | 30 days | Remained yellow solid powder; moisture content unchanged (0.67%). |
| NMNH-bis(L-ly sine) salt (1:2) | Amorphous (Yellow) | 30 days | Remained yellow solid powder; moisture content unchanged (0.74%). |
| NMNH-L-lysine salt (1:1) | Amorphous (Yellow) | 30 days | Remained yellow solid powder; moisture content unchanged (0.79%). |
| NMNH-bis(L-hi stidine) salt (1:2) | Amorphous (Yellow) | 30 days | Remained yellow solid powder; moisture content unchanged (0.52%). |
| NMNH-L-histidi ne salt (1:1) | Amorphous (Yellow) | 30 days | Remained yellow solid powder; moisture content unchanged (0.59%). |

From the data in the table above, it can be seen that:
The amorphous NMNH-K₂ salt exhibited similar properties to the amorphous NMNH-Na₂ salt reported in WO2023160405A1. Upon open placement at 25°C and 65% RH for 1 day, the powder absorbed moisture and turned oily, with purity decreasing from 99.61% to 99.24%.

The NMNH-Mg salt, upon open placement at 25°C and 65% RH for 30 days, remained as a yellow solid powder with unchanged purity (99.53%) and unchanged moisture content (0.23%). The NMNH-Na₂ salt crystalline Form A reported in WO2023160405A1 had a moisture content as high as 19%-30%. Compared with the NMNH-Na₂ salt, the amorphous NMNH-Mg salt exhibited better stability, lower moisture content, and stronger oxidation resistance and moisture resistance, demonstrating significantly superior performance.

The NMNH-Zn salt, NMNH-Fe(III) salt, NMNH-Cu(II) salt, and NMNH-Mn(II) salt, upon open placement at 25°C and 65% RH for 30 days, all maintained their appearance, color, and moisture content unchanged, and exhibited higher stability, oxidation resistance, and moisture resistance than the NMNH-Na₂ salt as well.

NMNH-bis(D-glucosamine) salt (1:2), NMNH-D-glucosamine salt (1:1), NMNH-bis(L-arginine) salt (1:2), NMNH-L-arginine salt (1:1), NMNH-bis(L-lysine) salt (1:2), NMNH-L-lysine salt (1:1), NMNH-bis(L-histidine) salt (1:2), and NMNH-L-histidine salt (1:1) all belong to organic salts of NMNH (salts formed with organic matters). Compared with inorganic salts (Na, K, etc.), these salt-forming organic compounds possess a certain degree of hydrophobicity, thus exhibiting stronger moisture resistance, consistent with the data shown in the table above.

All references mentioned in the present invention are incorporated herein by reference as if each individual reference was incorporated by reference. In addition, it should be understood that after reading the above teachings of the present invention, those skilled in the art can make various changes or modifications to the present invention, and these equivalent forms also fall within the scope defined by the appended claims of the present application.

## Claims

1. A reduced β-nicotinamide mononucleotide salt-type compound (NMNH-M) represented by formula (I): 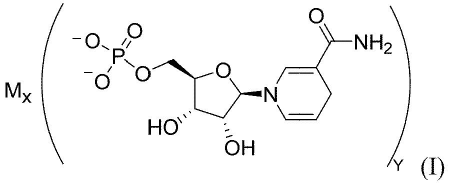 wherein,
M is independently selected from the group consisting of: Mg²⁺, Zn²⁺, Fe³⁺, Cu²⁺, Mn²⁺, and an organic matter;
when M is Mg²⁺, X=1, Y=1;
when M is Zn²⁺, X=1, Y=1;
when M is Fe³⁺, X=2, Y=3;
when M is Cu²⁺, X=1, Y=1;
when M is Mn²⁺, X=1, Y=1;
wherein the organic matter is selected from the group consisting of: D-glucosamine, L-arginine, L-lysine and L-histidine.

2. The compound according to claim 1, **characterized in that** the compound is amorphous.

3. A method for preparing the compound according to claim 1, **characterized in that** it comprises steps of:
(1) adding reduced β-nicotinamide mononucleotide disodium salt to a first solvent, stirring and adjusting pH to 3-4 to obtain solution A, or to *in situ* generate reduced β-nicotinamide mononucleotide solution A through a reaction;
(2) adding a solution of an inorganic salt, an organic matter, or an organic salt to solution A obtained in step (1), adjusting pH, and stirring for reaction to obtain solution B;
(3) removing the solvent from solution B obtained in step (2) and drying to obtain the reduced β-nicotinamide mononucleotide salt-type compound.

4. The method according to claim 3, **characterized in that** the organic matter is selected from D-glucosamine, L-arginine, L-lysine and L-histidine.

5. The method according to claim 3, **characterized in that** the molar ratio of the reduced β-nicotinamide mononucleotide to the corresponding organic matter is 1:1-2.5, preferably 1:1-2.

6. A pharmaceutical composition, comprising the reduced β-nicotinamide mononucleotide salt-type compound according to claim 1 or a pharmaceutically acceptable carrier thereof.

7. A method for preparing a pharmaceutical composition, **characterized in that** the method comprises a step of:
mixing the reduced β-nicotinamide mononucleotide salt-type compound according to claim 1 with a pharmaceutically acceptable carrier to form the pharmaceutical composition.

8. Use of the reduced β-nicotinamide mononucleotide salt-type compound according to claim 1 or the pharmaceutical composition according to claim 6, **characterized in that** it is used for the preparation of a product for elevating nicotinamide adenine dinucleotide (NAD⁺) to delay aging, and/or for preventing or treating diseases or symptoms caused by or resulting from NAD⁺ deficiency.

9. Use of the reduced β-nicotinamide mononucleotide salt-type compound according to claim 1 or the pharmaceutical composition according to claim 6, **characterized in that** it is used for the preparation of a product for preventing and/or treating diseases or symptoms caused by or resulting from deficiency of human essential elements.

10. Use of the reduced β-nicotinamide mononucleotide salt-type compound according to claim 1 or the pharmaceutical composition according to claim 6, **characterized in that** it is used for the preparation of a product for preventing and/or treating diseases or symptoms caused by or resulting from conditions such as malnutrition, loss of appetite, and developmental insufficiency.
